# EUROPEAN PATENT SPECIFICATION

(11) **EP 2 245 062 B1**
(45) Date of publication and mention of the grant of the patent: **25.04.2012**
(21) Application number: 09702831.0
(22) Date of filing: 15.01.2009
(51) Int. Cl.: C07K 16/24, A61K 39/395, A61P 19/10

(54) **METHODS OF TREATING BONE-LOSS DISORDERS USING A GM-CSF ANTAGONIST**
VERFAHREN ZUR BEHANDLUNG VON ERKRANKUNGEN MIT KNOCHENABBAU UNTER VERWENDUNG EINES GM-CSF-ANTAGONISTEN
PROCÉDÉS DE TRAITEMENT DE TROUBLES DE LA PERTE OSSEUSE EN UTILISANT UN ANTAGONISTE GM-CSF

(30) Priority: 15.01.2008 US 21218 P
(43) Date of publication of application: 03.11.2010
(73) Proprietor: Kalobios Pharmaceuticals, Inc., South San Francisco, CA 94080 (US)
(72) Inventor: BEBBINGTON, Christopher R., South San Francisco California 94080 (US); YARRANTON, Geoffrey T., South San Francisco California 94080 (US)
(74) Representative: Campbell, Patrick John Henry
(86) International application number: PCT/US2009/031126
(87) International publication number: WO 2009/091905

(56) References cited:
- WO-A-03/068920
- WO-A-2008/064321
- US-A1- 2002 141 994
- US-B1- 6 372 739
- PARK BAE KEUN ET AL: "NF-kappa B in breast cancer cells promotes osteolytic bone metastasis by inducing osteoclastogenesis via GM-CSF" NATURE MEDICINE, NATURE PUBLISHING GROUP, NEW YORK, NY, US, vol. 13, no. 1, 1 January 2007 (2007-01-01), pages 62-69, XP009117859 ISSN: 1078-8956
- SUTHERLAND MAY S KUNG ET AL: "SP500263, a novel SERM, blocks osteoclastogenesis in a human bone cell model: Role of IL-6 and GM-CSF" CYTOKINE, ACADEMIC PRESS LTD, PHILADELPHIA, PA, US, vol. 23, no. 1-2, 1 July 2003 (2003-07-01), pages 1-14, XP009117858 ISSN: 1043-4666
- PINKERTON ET AL: "Combination therapy for treatment of osteoporosis: A review" AMERICAN JOURNAL OF OBSTETRICS & GYNECOLOGY, MOSBY, ST LOUIS, MO, US, vol. 197, no. 6, 3 December 2007 (2007-12-03), pages 559-565, XP022366670 ISSN: 0002-9378
- BLAHOS JAROSLAV: "Treatment and prevention of osteoporosis." WIENER MEDIZINISCHE WOCHENSCHRIFT (1946) 2007, vol. 157, no. 23-24, 2007, pages 589-592, XP019568250 ISSN: 0043-5341
- "PREVENTION AND MANAGEMENT OF OSTEOPOROSIS" WHO TECHNICAL REPORT SERIES,, vol. 921, 1 January 2003 (2003-01-01), pages 1-206, XP009117950
- MAKINEN TATU J ET AL: "The incidence of osteopenia and osteoporosis in women with hip osteoarthritis scheduled for cementless total joint replacement" BONE (NEW YORK), vol. 40, no. 4, April 2007 (2007-04), pages 1041-1047, XP009117954 ISSN: 8756-3282
- KHAPLI SHRUTI M. ET AL: 'IL-3 acts directly on osteoclast precursors and irreversibly inhibits receptor activator of NF-kappa B ligand-induced osteoclast differentiation by diverting the cells to macrophage lineage.' JOURNAL OF IMMUNOLOGY (BALTIMORE, MD. : 1950) 1 JUL 2003 LNKD- PUBMED:12816992 vol. 171, no. 1, 01 July 2003, pages 142 - 151 ISSN: 0022-1767
- MIYAMOTO T. ET AL: 'Bifurcation of osteoclasts and dendritic cells from common progenitors.' BLOOD 15 OCT 2001 LNKD- PUBMED:11588053 vol. 98, no. 8, 15 October 2001, pages 2544 - 2554 ISSN: 0006-4971
- MYINT Y.Y. ET AL: 'Granulocyte/macrophage colony-stimulating factor and interleukin-3 correct osteopetrosis in mice with osteopetrosis mutation.' THE AMERICAN JOURNAL OF PATHOLOGY FEB 1999 LNKD- PUBMED:10027413 vol. 154, no. 2, February 1999, pages 553 - 566 ISSN: 0002-9440
- SHUTO T. ET AL: 'Dexamethasone stimulates osteoclast-like cell formation by inhibiting granulocyte-macrophage colony-stimulating factor production in mouse bone marrow cultures.' ENDOCRINOLOGY MAR 1994 LNKD- PUBMED:8119150 vol. 134, no. 3, March 1994, pages 1121 - 1126 ISSN: 0013-7227
- KIM MICHAEL S. ET AL: 'MCP-1 is induced by receptor activator of nuclear factor-{kappa}B ligand, promotes human osteoclast fusion, and rescues granulocyte macrophage colony-stimulating factor suppression of osteoclast formation.' THE JOURNAL OF BIOLOGICAL CHEMISTRY 22 APR 2005 LNKD- PUBMED:15722361 vol. 280, no. 16, 22 April 2005, pages 16163 - 16169 ISSN: 0021-9258

## Description

### CROSS-REFERENCE TO RELATED APPLICATIONS

This applications claims benefit of U.S. provisional application no. 61/021,218 filed January 15, 2008.

### BACKGROUND OF THE INVENTION

Osteoporosis is a common condition in aging women and men where loss of bone, disruption of bone microarchitecture, and changes in non-collagenous proteins in bone lead to increased risk of fracture. About 25 to 30 percent of all white females in the United States develop symptomatic osteoporosis. A direct relationship exists between osteoporosis and the incidence of hip, femoral, neck and inter-trochanteric fracture in women 45 years and older. Elderly males develop symptomatic osteoporosis between the ages of 50 and 70, but the disease primarily affects females.

A primary cause of osteoporosis is an imbalance of bone remodeling in which bone resorption predominates. In normal bone, there is constant matrix remodeling of bone. Bone is resorbed by osteoclasts, which derive from the bone marrow; new bone is deposited by osteoblasts. Bone re-modeling is influenced by a variety of hormonal factors. For example, the activation of osteoclasts is regulated by number of factors, including osteoprotegerin (OPG) and Receptor Activator for Nuclear Factor κB Ligand (RANKL). RANKL increases bone resorption upon binding to its receptor. OPG that binds to RANKL can suppress its ability to increase bone resorption by inhibiting binding to the receptor. Various other cytokines and factors regulate bone resorption by influencing proliferation, maturation and secretory activities of bone cells and bone cell precursors. These cytokines and growth factors include IL-6, TNF-a, IL-1 and M-CSF. In particular, US 6372739 describes estrogen antagonists and/or agonists that block production of IL-6 and/or GM-CSF and inhibit osteoclast formation in an *in vitro* culture system.

However, there is a considerable body of evidence that GM-CSF inhibits osteoclast differentiation by converting precursors into dendritic cells (*see, e.g.,* Khapli et al, J. Immunol. 171:142-151, 2003; Miyamoto et al, Blood 98:2544-2554, 2001; Myint et al., Am. J. Pathol. 154:553-566, 1999; Shuto et al, Endocrinology 134:1121-1126, 1994; and Kim et al, J. Biol. Chem. 280:16163-16169, 2005). There have also been reports that under certain conditions, GM-CSF may promote the formation of osteoclastic cells *in vitro* (e.g., U.S. Patent No. 6,331,562) and that colony stimulating factors may be therapeutic targets in particular circumstances (U.S. Patent Application Publication No. 20020141994). Thus, the role of GM-CSF in osteoporosis has not been clearly delineated.

This invention addresses the need for additional therapies for osteoporosis and other disorders where bone density is lost by providing new methods of treating osteoporosis that target GM-CSF.

### BRIEF SUMMARY OF THE INVENTION

The present invention is based on the discovery that a GM-CSF antagonist can be used to treat bone loss disorders such as osteopenia. Thus, the invention provides a neutralizing anti-GM-CSF antibody for use in a method for treating a patient that has osteopenia that is a general loss of bone density below normal that is not site-specific, the method comprising administering a therapeutically effective amount of said antibody to the patient in an amount sufficient to reduce the symptoms of bone loss, wherein said antibody binds to GM-CSF with a dissociation constant (K_{D}) less than about 100 pM.

In some embodiments, the GM-CSF antibody of the invention is recombinantly produced, *e.g*., a recombinant monoclonal antibody. In other embodiments, the GM-CSF antibody of the invention, *e.g*., purified anti-GM-CSF from human plasma, is purified from a natural source.

In some embodiments, the patient having osteopenia has osteoporosis. In typical embodiments, the ostepenia is due to estrogen depletion. In some embodiments, a GM-CSF antibody of the invention is administered to patient diagnosed with osteopenia that is a general loss of bone density below normal that is not site-specific, *e.g*., osteoporosis, or another disorder involving bone loss, where the patient has also been diagnosed with other disease, *e.g*., an autoimmune disease such as rheumatoid arthritis. In other embodiments, the GM-CSF antibody of the invention, is administered to a patient that has been diagnosed with osteopenia that is a general loss of bone density below normal that is not site-specific, e.g., osteoporosis, and has not been diagnosed with rheumatoid arthritis; or to a patient that has not been diagnosed with an autoimmune disease.

In various embodiments, the antibody can be a polyclonal antibody, a monoclonal antibody, or an antibody such as a nanobody or a camelid antibody. In some embodiments, the antibody is an antibody fragment, such as a Fab, a Fab', a F(ab')₂, a scFv, or a domain antibody (dAB). The antibody can also be modified, *e.g*., to enhance stability. Thus, in some embodiments, the antibody is conjugated to polyethylene glycol.

In further embodiments, the antibody has an affinity of about 1 pM to about 100 pM, e.g., an affinity of about 1 pM, about 5 pM, about 10 pM, about 15 pM, about 20 pM, about 25 pM, about 30 pM, about 40 pM, about 50 pM, about 60 pM, about 70 pM, about 80 pM, or about 90 pM to about 100 pM. In some embodiments, the antibody has an affinity of from about 10 to about 30 pM.

In further embodiments, the antibody is a recombinant or chimeric antibody. In some embodiments, the antibody is a human antibody. In some embodiments, the antibody comprises a human variable region. In some embodiments, the antibody comprises a human light chain constant region. In some embodiments, the antibody comprises a human heavy chain constant region, such as a gamma chain.

In further embodiments, the antibody binds to the same epitope as a chimeric 19/2 antibody. The antibody can, e.g., comprise the V_{H} and V_{L} regions of chimeric 19/2. The antibody can also comprise a human heavy chain constant region such as a gamma region. In some embodiments, the antibody comprises the CDR1, CDR2, and CDR3 of the V_{H} region of chimeric 19/2. In further embodiments, the antibody comprises the CDR1, CDR2, and CDR3 of the V_{L} region of chimeric 19/2. In additional embodiments, the antibody comprises the CDR1, CDR2, and CDR3 of the V_{H} and V_{L} regions of a chimeric 19/2 antibody. In some embodiments, the antibody comprises the V_{H} region CDR3 and V_{L} region CDR3 of chimeric 19/2.

In some embodiments, the antibody has a half-life of about 7 to about 25 days.

In some embodiments of the invention, the anti-GM-CSF antibody is administered by injection or by infusion. For example, the GM-CSF antibody can be administered intravenously over a period between about 15 minutes and about 2 hours.

In other embodiments, the GM-CSF antibody of the invention is administered subcutaneously by bolus injection.

In further embodiments, the GM-CSF antibody of the invention is administered intramuscularly.

A GM-CSF antibody can, for example, be administered at a dose between about 1 mg/kg of body weight and about 10 mg/kg of body weight.

In some embodiments, treatment with the GM-CSF antibody of the invention comprises a second administration of the GM-CSF antibody.

In some embodiments, the treatment methods further comprise administering a second therapeutic agent for the treatment of bone loss, *e.g*., a bisphosphonate such as alendronate, etidronate, risedronate or ibandronic acid; or an agent such as raloxifene, teriparatide, or strontium ranelate; or a bone-enhancing mineral such as fluoride or calcium. Other therapeutic agents include calcitonin, hormone replacement therapy, RANKL antagonists such as osteoprotegerin (OPG), *e.g*., OPG-Fc fusion proteins, and antibodies to RANKL, *e.g*., denosumab. The GM-CSF antibody, may be administered concomitantly or sequentially with the one or more of the desired additional therapeutic agents. In some embodiments, a patient may initially be treated with an agent, *e.g*., a bisphosphonate, and then receive treatment with the GM-CSF antibody after treatment with the bisphosphonate has been discontinued. In some embodiments, a lower dose, and/or less frequent dosages, of an additional therapeutic agent, *e.g*., a bisphosphonate, may be used when the patient also undergoes treatment with a GM-CSF antibody, in comparison to the amount of therapeutic agent, *e.g*., a bisphosphonate, typically administered to a patient in the absence of treatment with a GM-CSF antibody.

### BRIEF DESCRIPTION OF THE DRAWINGS

Figure 1 shows treatment groups in a study evaluating the effects of a GM-CSF antibody on bone loss in a mouse model of osteoporosis. Routes of administration are intraperitoneal (IP) or subcutaneous (SC). (NA: not applicable)

Figure 2 shows data analyzing canellous bone density. Figure 2 shows inhibition of ovariectomy-induced osteopenia in mice: histomorphometric analysis of canellous bone density

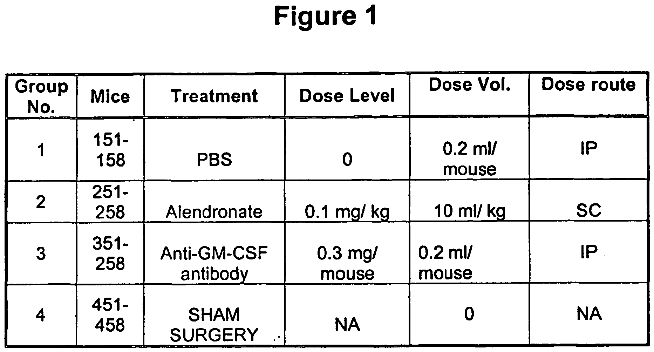

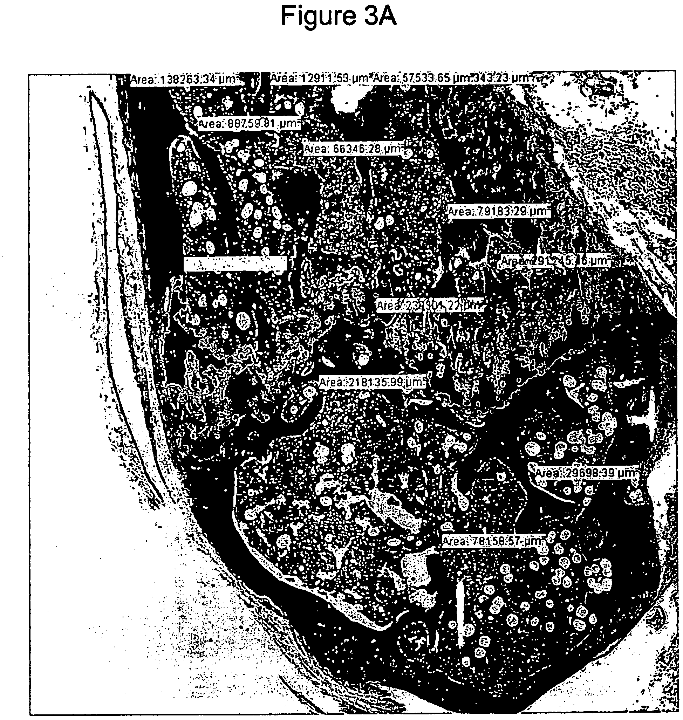

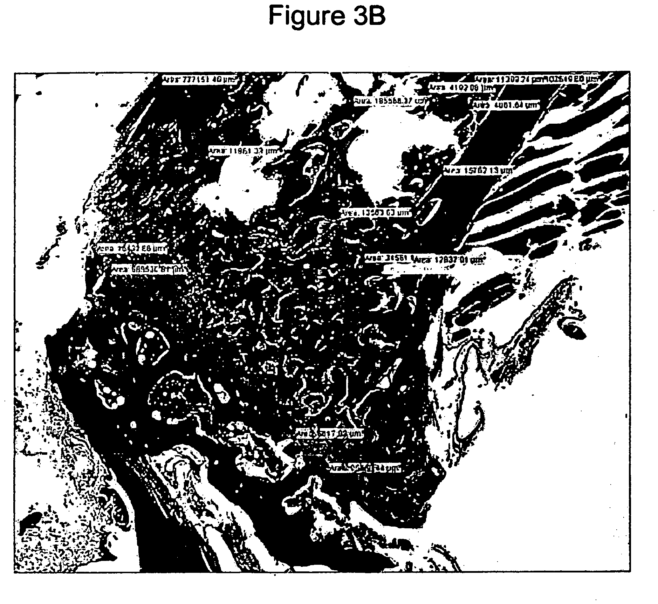

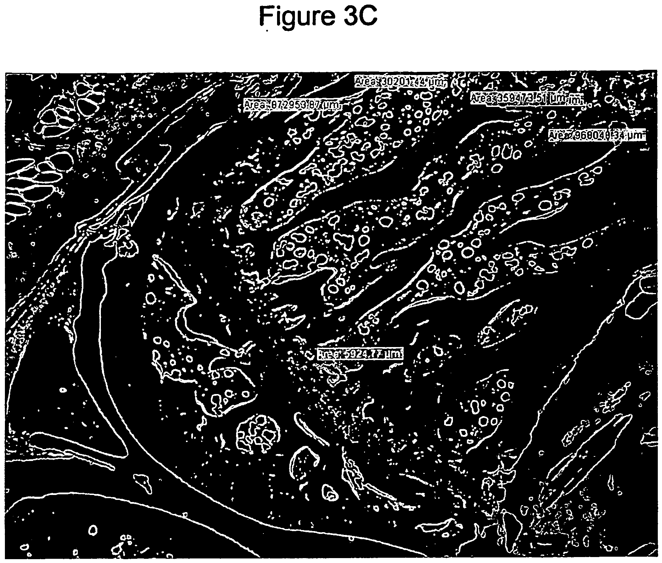

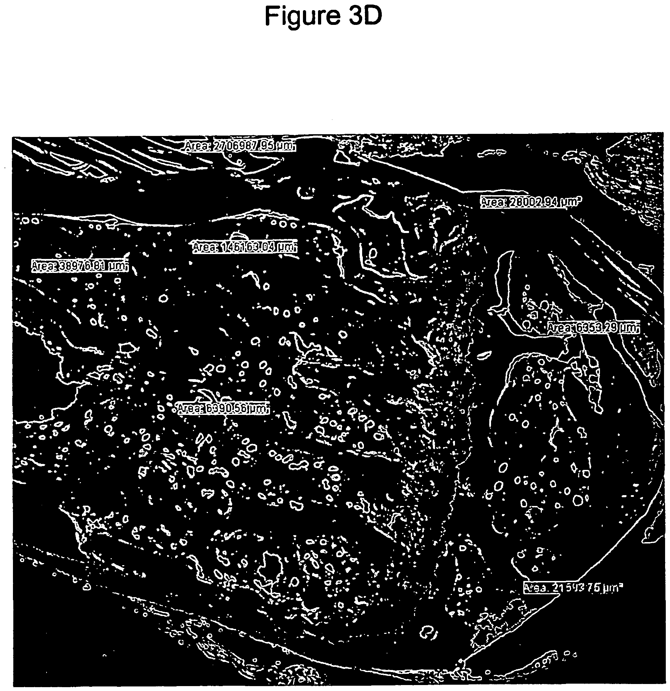

Figure 3 shows photomicrographs of sections of the epiphyseal area of decalcified tibia, stained with hematoxylin and eosin (H&E) from mice treated as shown in Figure 1. Figure 3A) a control-treated mouse (group 1) demonstrating the reduction in size, number and density of trabeculae in a mouse with ovariectomy-induced osteopenia; Figure 3B) an ovariectomized mouse treated with alendronate (group 2) demonstrating increase in size, number and density of trabeculae; Figure 3C) an ovariectomized mouse treated with anti-GM-CSF antibody (group 3) demonstrating increase in size, number and density of trabeculae; Figure 3D) an animal in group 4 (sham surgery) demonstrating normality in size, number and density of trabeculae.

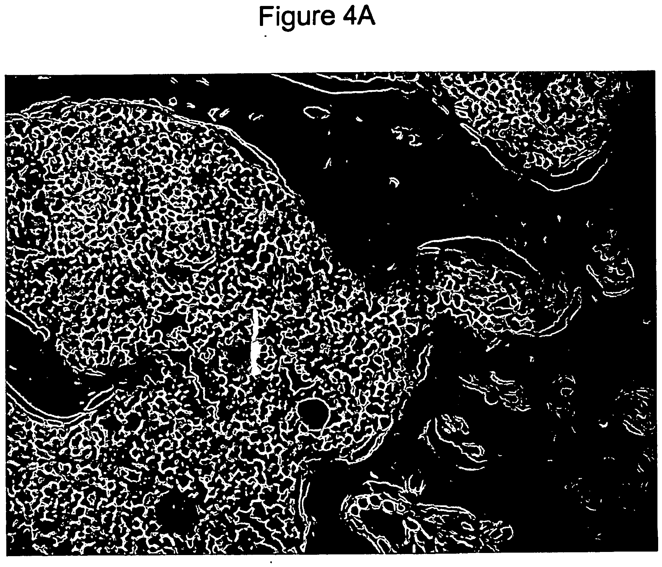

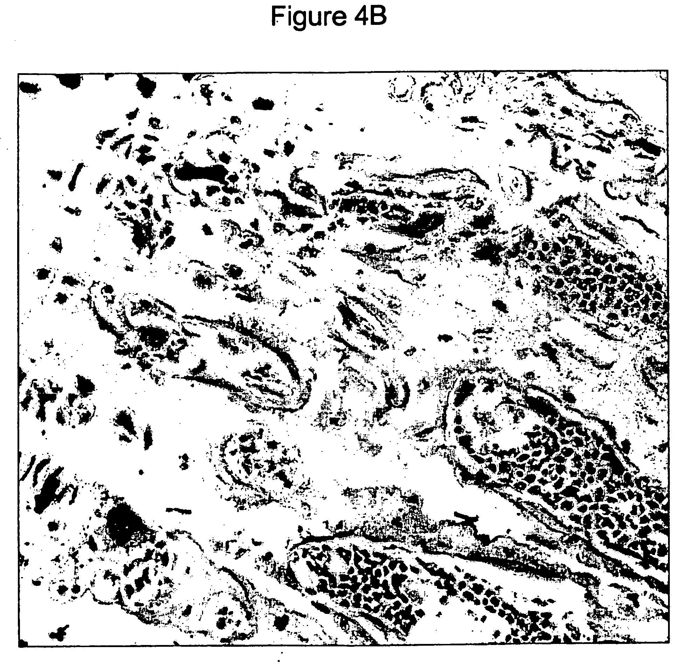

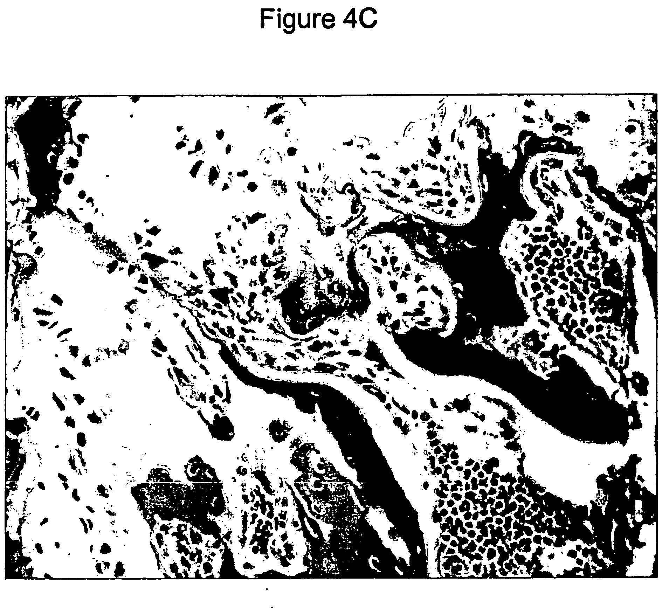

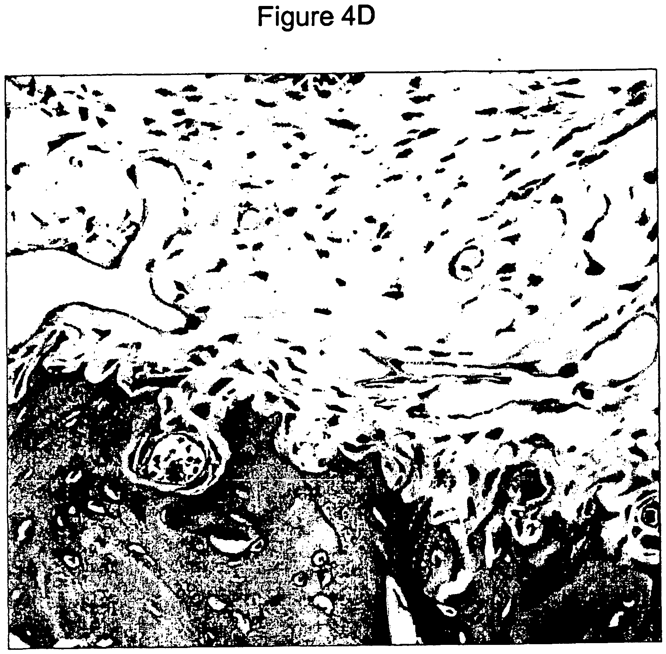

Figure 4 shows photomicrographs stained with hematoxylin and eosin (H&E) depicting osteoblast and osteoclast morphology and activity in sections of decalcified bone from ovariectomized and sham-operated mice. Figure 4A). Illustration of osteoblasts and osteoclasts morphology and activity in an animal from group 1 (ovariectomized; control treated); Figure 4B) Illustration of osteoblasts and osteoclasts morphology and activity in an animal from group 2 (ovariectomized; treated with alendronate); Figure 4C) Illustration of osteoblasts and osteoclasts morphology and activity in an animal from group 3 (ovariectomized and treated with anti-GM-CSF antibody); Figure 4D) Illustration of osteoblasts and osteoclasts morphology and activity in an animal from group 4 (sham-operated).

Figures 5 shows hematological comparisons of animals treated with GM-CSF antibody to normal and ovariectomized animals that do not receive the antibody.

### DETAILED DESCRIPTION OF THE INVENTION

### Definitions

As used herein, a "bone loss disorder" refers to a loss of bone density, either localized or non-specific. "Osteopenia" in the context of this invention refers to general loss of bone density below normal, where the bone loss is not site-specific. "Osteoporosis" is a type of osteopenia where bone loss is more advanced and is diagnosed based on common clinical standards.

As used herein, "Granulocyte Macrophage-Colony Stimulating Factor" (GM-CSF) refers to a small a naturally occurring glycoprotein with internal disulfide bonds having a molecular weight of approximately 23 kDa. In humans, it is encoded by a gene located within the cytokine cluster on human chromosome 5. The sequence of the human gene and protein are known. The protein has an N-terminal signal sequence, and a C-terminal receptor binding domain (Rasko and Gough In: The Cytokine Handbook, A. Thomson, et al, Academic Press, New York (1994) pages 349-369). Its three-dimensional structure is similar to that of the interleukins, although the amino acid sequences are not similar. GM-CSF is produced in response to a number of inflammatory mediators by mesenchymal cells present in the hemopoietic environment and at peripheral sites of inflammation. GM-CSF is able to stimulate the production of neutrophilic granulocytes, macrophages, and mixed granulocyte-macrophage colonies from bone marrow cells and can stimulate the formation of eosinophil colonies from fetal liver progenitor cells. GM-CSF can also stimulate some functional activities in mature granulocytes and macrophages.

The term "granulocyte macrophage-colony stimulating factor receptor" (GM-CSFR)" refers to a membrane bound receptor expressed on cells that transduces a signal when bound to granulocyte macrophage colony-stimulating factor (GM-CSF). GM-CSFR consists of a ligand-specific low-affinity binding chain (GM-CSFR alpha) and a second chain that is required for high-affinity binding and signal transduction. This second chain is shared by the ligand-specific alpha-chains for the interleukin 3 (IL-3) and IL-5 receptors and is therefore called beta common (beta c). The cytoplasmic region of GM-CSFR alpha consists of a membrane-proximal conserved region shared by the alpha 1 and alpha 2 isoforms and a C-terminal variable region that is divergent between alpha 1 and alpha 2. The cytoplasmic region of beta-c contains membrane proximal serine and acidic domains that are important for the proliferative response induced by GM-CSF

The term "soluble granulocyte macrophage-colony stimulating factor receptor" (sGM-CSFR) refers to a non-membrane bound receptor that binds GM-CSF, but does not transduce a signal when bound to the ligand.

As used herein, "GM-CSF antagonist" refers to a molecule or compound that interacts with GM-CSF, or its receptor, to reduce or block (either partially or completely) signal transduction that would otherwise result from the binding of GM-CSF to its cognate receptor expressed on cells. GM-CSF antagonists may act by reducing the amount of GM-CSF ligand available to bind the receptor (e.g., antibodies that once bound to GM-CSF increase the clearance rate of GM-CSF) or prevent the ligand from binding to its receptor either by binding to GM-CSF or the receptor (e.g., neutralizing antibodies). GM-CSF antagonist may also include inhibitors, which may include compounds that bind GM-CSF or its receptor to partially or completely inhibit signaling. GM-CSF antagonist may include antibodies, natural or synthetic ligands or fragments thereof, polypeptides, small molecules, and the like.

A "purified" GM-CSF antagonist as used herein refers to a GM-CSF antagonist that is substantially or essentially free from components that normally accompany it as found in its native state. For example, a GM-CSF antagonist such as an anti-GM-CSF antibody, that is purified from blood or plasma is substantially free of other blood or plasma components such as other immunoglobulin molecules. Purity and homogeneity are typically determined using analytical chemistry techniques such as polyacrylamide gel electrophoresis or high performance liquid chromatography. A protein that is the predominant species present in a preparation is substantially purified. Typically, "purified" means that the protein is at least 85% pure, more preferably at least 95% pure, and most preferably at least 99% pure relative to the components with which the protein naturally occurs.

As used herein, an "antibody" refers to a protein functionally defined as a binding protein and structurally defined as comprising an amino acid sequence that is recognized by one of skill as being derived from the framework region of an immunoglobulin-encoding gene of an animal that produces antibodies. An antibody can consist of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains, respectively.

The term antibody as used herein includes antibody fragments that retain binding specificity. For example, there are a number of well characterized antibody fragments. Thus, for example, pepsin digests an antibody C-terminal to the disulfide linkages in the hinge region to produce F(ab)'₂, a dimer of Fab which itself is a light chain joined to VH-CH1 by a disulfide bond. The F(ab)'₂ may be reduced under mild conditions to break the disulfide linkage in the hinge region thereby converting the (Fab')₂ dimer into an Fab' monomer. The Fab' monomer is essentially a Fab with part of the hinge region (see, Fundamental Immunology, W.E. Paul, ed., Raven Press, N.Y. (1993), for a more detailed description of other antibody fragments). While various antibody fragments are defined in terms of the digestion of an intact antibody, one of skill will appreciate that fragments can be synthesized de novo either chemically or by utilizing recombinant DNA methodology. Thus, the term antibody also includes antibody fragments either produced by the modification of whole antibodies or synthesized using recombinant DNA methodologies.

Antibodies include dimers such as V_{H}-V_{L} dimers, V_{H} dimers, or V_{L} dimers, including single chain antibodies (antibodies that exist as a single polypeptide chain), such as single chain Fv antibodies (sFv or scFv) in which a variable heavy and a variable light region are joined together (directly or through a peptide linker) to form a continuous polypeptide. The single chain Fv antibody is a covalently linked V_{H}-V_{L} heterodimer which may be expressed from a nucleic acid including V_{H}- and V_{L}- encoding sequences either joined directly or joined by a peptide-encoding linker (*e.g*., Huston, et al. Proc. Nat. Acad. Sci. USA, 85:5879-5883, 1988). While the V_{H} and V_{L} are connected to each as a single polypeptide chain, the V_{H} and V_{L} domains associate non-covalently. Alternatively, the antibody can be another fragment, such as a disulfide-stabilized Fv (dsFv). Other fragments can also be generated, including using recombinant techniques. The scFv antibodies and a number of other structures converting the naturally aggregated, but chemically separated light and heavy polypeptide chains from an antibody V region into a molecule that folds into a three dimensional structure substantially similar to the structure of an antigen-binding site are known to those of skill in the art (see e.g., U.S. Patent Nos. 5,091,513, 5,132,405, and 4,956,778). In some embodiments, antibodies include those that have been displayed on phage or generated by recombinant technology using vectors where the chains are secreted as soluble proteins, *e.g*., scFv, Fv, Fab, (Fab')₂ or generated by recombinant technology using vectors where the chains are secreted as soluble proteins. Antibodies for use in the invention can also include diantibodies and miniantibodies.

Antibodies of the invention also include heavy chain dimers, such as antibodies from camelids. Since the V_{H} region of a heavy chain dimer IgG in a camelid does not have to make hydrophobic interactions with a light chain, the region in the heavy chain that normally contacts a light chain is changed to hydrophilic amino acid residues in a camelid. V_{H} domains of heavy-chain dimer IgGs are called VHH domains. Antibodies for use in the current invention include single domain antibodies (dAbs) and nanobodies (*see, e.g.,* Cortez-Retamozo, et al., Cancer Res. 64:2853-2857, 2004).

As used herein, "V-region" refers to an antibody variable region domain comprising the segments of Framework 1, CDR1, Framework 2, CDR2, and Framework 3, including CDR3 and Framework 4, which segments are added to the V-segment as a consequence of rearrangement of the heavy chain and light chain V-region genes during B-cell differentiation.

As used herein, "complementarity-determining region (CDR)" refers to the three hypervariable regions in each chain that interrupt the four "framework" regions established by the light and heavy chain variable regions. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, for example, a V_{H} CDR3 is located in the variable domain of the heavy chain of the antibody in which it is found, whereas a V_{L} CDR1 is the CDR1 from the variable domain of the light chain of the antibody in which it is found.

The sequences of the framework regions of different light or heavy chains are relatively conserved within a species. The framework region of an antibody, that is the combined framework regions of the constituent light and heavy chains, serves to position and align the CDRs in three dimensional space.

The amino acid sequences of the CDRs and framework regions can be determined using various well known definitions in the art, *e.g*., Kabat, Chothia, international ImMunoGeneTics database (IMGT), and AbM (*see, e.g.,* Johnson *et al*., *supra*; Chothia & Lesk, 1987, Canonical structures for the hypervariable regions of immunoglobulins. J. Mol. Biol. 196, 901-917; Chothia C. et al., 1989, Conformations of immunoglobulin hypervariable regions. Nature 342, 877-883; Chothia C. et al., 1992, structural repertoire of the human VH segments J. Mol. Biol. 227, 799-817; Al-Lazikani et al., J.Mol.Biol 1997, 273(4)). Definitions of antigen combining sites are also described in the following: Ruiz et al., IMGT, the international ImMunoGeneTics database. Nucleic Acids Res., 28, 219-221 (2000); and Lefranc,M.-P. IMGT, the international ImMunoGeneTics database. Nucleic Acids Res. Jan 1;29(1):207-9 (2001); MacCallum et al, Antibody-antigen interactions: Contact analysis and binding site topography, J. Mol. Biol., 262 (5), 732-745 (1996); and Martin et al, Proc. Natl Acad. Sci. USA, 86, 9268-9272 (1989); Martin, et al, Methods Enzymol., 203, 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols in Methods in Molecular Biology, Vol. 66, Glenn E. Morris, Ed (1996).

As used herein, "neutralizing antibody" refers to an antibody that binds to GM-CSF and prevents signaling by the GM-CSF receptor, or inhibits binding of GM-CSF to its receptor.

As used herein, "chimeric antibody" refers to an immunoglobulin molecule in which (a) the constant region, or a portion thereof, is altered, replaced or exchanged so that the antigen binding site (variable region) is linked to a constant region of a different or altered class, effector function and/or species, or an entirely different molecule that confers new properties to the chimeric antibody, e.g., an enzyme, toxin, hormone, growth factor, drug, etc.; or (b) the variable region, or a portion thereof, is altered, replaced or exchanged with a variable region, or portion thereof, having a different or altered antigen specificity; or with corresponding sequences from another species or from another antibody class or subclass.

As used herein, "humanized antibody" refers to an immunoglobulin molecule in which the CDRs from a donor antibody are grafted onto human framework sequences. Humanized antibodies may also comprise residues of donor origin in the framework sequences. The humanized antibody can also comprise at least a portion of a human immunoglobulin constant region. Humanized antibodies may also comprise residues which are found neither in the recipient antibody nor in the imported CDR or framework sequences. Humanization can be performed using methods known in the art (*e.g*., Jones et al., Nature 321:522-525; 1986; Riechmann et al., Nature 332:323-327, 1988; Verhoeyen et al., Science 239:1534-1536, 1988); Presta, Curr. Op. Struct. Biol. 2:593-596, 1992; U.S. Patent No. 4,816,567), including techniques such as "superhumanizing" antibodies (Tan et al., J. Immunol. 169: 1119, 2002) and "resurfacing" (*e.g*., Staelens et al., Mol. Immunol. 43: 1243, 2006; and Roguska et al., Proc. Natl. Acad. Sci USA 91: 969, 1994).

A "humaneered" antibody in the context of this invention refers to an engineered human antibody having a binding specificity of a reference antibody. A "humaneered" antibody for use in this invention has an immunoglobulin molecule that contains minimal sequence derived from a donor immunoglobulin. Typically, an antibody is "humaneered" by joining a DNA sequence encoding a binding specificity determinant (BSD) from the CDR3 region of the heavy chain of the reference antibody to human V_{H} segment sequence and a light chain CDR3 BSD from the reference antibody to a human V_{L} segment sequence. A "BSD" for a CDR3 can thus refer to a CDR3-FR4 region, or a portion of this region that mediates binding specificity. A binding specificity determinant therefore can be a CDR3-FR4, a CDR3, a minimal essential binding specificity determinant of a CDR3 (which refers to any region smaller than the CDR3 that confers binding specificity when present in the V region of an antibody), the D segment (with regard to a heavy chain region), or other regions of CDR3-FR4 that confer the binding specificity of a reference antibody. Methods for humaneering are provided in US patent application publication no. 20050255552 and US patent application publication no. 20060134098.

The term "binding specificity determinant" or "BSD" as used in the context of this invention refers to the minimum contiguous or non-contiguous amino acid sequence within a CDR region necessary for determining the binding specificity of an antibody.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid. Similarly, a heterologous protein will often refer to two or more subsequences that are not found in the same relationship to each other in nature.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed in vitro, in general, by the manipulation of nucleic acid, e.g., using polymerases and endonucleases, in a form not normally found in nature. In this manner, operably linkage of different sequences is achieved. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention. Similarly, a "recombinant protein" is a protein made using recombinant techniques, i.e., through the expression of a recombinant nucleic acid as depicted above.

The phrase "specifically (or selectively) binds" to an antibody or "specifically (or selectively) immunoreactive with," when referring to a protein or peptide, refers to a binding reaction where the antibody binds to the protein of interest. An antibody typically binds to a protein or peptide with an affinity of 500 nM or less, and has an affinity of 5000 nM or greater, for other antigens.

As used herein, "a therapeutic agent for the treatment of a bone loss disorder" refers to an agent that when administered to a patient suffering from a bone loss disorder such as osteoporosis in a therapeutically effective dose, at least partially arrests the loss of bone density and reduces or slows symptoms of the disorder and complications associated with the disorder.

The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (see, e.g., NCBI web site http://www.ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions, as well as naturally occurring, e.g., polymorphic or allelic variants, and man-made variants. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of one of the number of contiguous positions selected from the group consisting typically of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Current Protocols in Molecular Biology (Ausubel et al., eds. 1995 supplement)).

Preferred examples of algorithms that are suitable for determining percent sequence identity and sequence similarity include the BLAST and BLAST 2.0 algorithms, which are described in Altschul et al., Nuc. Acids Res. 25:3389-3402 (1977) and Altschul et al., J. Mol. Biol. 215:403-410 (1990). BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al*., *supra*). These initial neighborhood word hits act as seeds for initiating searches to find longer HSPs containing them. The word hits are extended in both directions along each sequence for as far as the cumulative alignment score can be increased. Cumulative scores are calculated using, e.g., for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

The BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001. Log values may be large negative numbers, e.g., 5, 10, 20, 30, 40, 40, 70, 90, 110, 150, 170, etc.

An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, e.g., where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequences.

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. A protein that is the predominant species present in a preparation is substantially purified. The term "purified" in some embodiments denotes that a protein gives rise to essentially one band in an electrophoretic gel. Preferably, it means that the protein is at least 85% pure, more preferably at least 95% pure, and most preferably at least 99% pure.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, e.g., an α carbon that is bound to a hydrogen, a carboxyl group, an amino group, and an R group, e.g., homoserine, norleucine, methionine sulfoxide, methionine methyl sulfonium. Such analogs may have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical or associated, e.g., naturally contiguous, sequences. Because of the degeneracy of the genetic code, a large number of functionally identical nucleic acids encode most proteins. For instance, the codons GCA, GCC, GCG and GCU all encode the amino acid alanine. Thus, at every position where an alanine is specified by a codon, the codon can be altered to another of the corresponding codons described without altering the encoded polypeptide. Such nucleic acid variations are "silent variations," which are one species of conservatively modified variations. Every nucleic acid sequence herein which encodes a polypeptide also describes silent variations of the nucleic acid. One of skill will recognize that in certain contexts each codon in a nucleic acid (except AUG, which is ordinarily the only codon for methionine, and TGG, which is ordinarily the only codon for tryptophan) can be modified to yield a functionally identical molecule. Accordingly, often silent variations of a nucleic acid which encodes a polypeptide is implicit in a described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. Typically conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

### I. Introduction

Described herein are methods of administering a GM-CSF antagonist for the treatment of patients that have a bone disorder in which bone density is lost, *e.g*., osteopenia. GM-CSF antagonists may include anti-GM-CSF antibodies, anti-GM-CSF receptor antibodies, or other inhibitors that prevent or reduce signaling that normally results from the binding of GM-CSF to its cognate receptor. Many types of GM-CSF antagonists are known (see, *e.g*., William, in New Drugs for Asthma, Allergy and COPD, Prog. Repir. Res.; Hansel & Barnes, eds, Basel, Karger, 2001 vol 31:251-255; and the reference cited therein).

Antibodies, *e.g*., anti-GM-CSF or anti-GM-CSF receptor antibodies, may be monoclonal, polyclonal, chimeric, humanized, humaneered, or human. Other GM-CSF antagonists may include naturally occurring or synthetic ligands (or fragments thereof) that compete with GM-CSF for binding to the receptor, but do not result in signaling when bound to the receptor. Additional non-limiting GM-CSF antagonists may include polypeptides, nucleic acids, small molecules and the like that either partially or completely block signaling that would naturally result from the binding of GM-CSF to its receptor in the absence of the GM-CSF antagonist.

### II. Patients With Bone Density Loss Disorders

A bone-loss disorder refers to a condition relating to any decrease in bone mass to below normal levels. Such a condition can occur due to a decrease in the rate of bone synthesis and/or an increase in the rate of bone break down. Osteopenia refers to a general loss of bone mass that is not site-specific. The most common form of osteopenia is primary osteoporosis This form of osteoporosis is a consequence of the universal loss of bone with age and is usually a result of increase in bone resorption with a normal rate of bone formation. Primary osteoporosis in women is often a result of loss of estrogen that occurs due to age-related menopause. However, osteoporosis may also occur in women who experience premature menopause as a result of genetics, illness, or medical procedures.

Other forms of bone loss include endocrine osteoporosis (*e.g*., due to hyperthyroidism, hyperparathyroidism, Cushing's syndrome, or acromegaly); hereditary and congenital forms of osteoporosis (osteogenesis imperfecta, homocystinuria, Menkes' syndrome, and Riley-Day syndrome); Paget's disease of bone (osteitis deformans) in adults and juveniles; osteomyelitis, or an infectious lesion in bone, leading to bone loss; and bone loss due to immobilization of extremities.

Bone loss may also induced by steroid administration, and associated with disorders of the small and large intestine and with chronic hepatic and renal diseases. Osteonecrosis, or bone cell death, associated with traumatic injury or nontraumatic necrosis associated with Gaucher's disease, sickle cell anemia, systemic lupus erythematosus and other conditions.

A bone loss disorder may be localized, *e.g*., resulting from surgery. Other forms of site-specific bone loss are periodontal bone loss, mandibular bone loss, and bone loss due to osteolytic metastasis.

Loss of bone density, *e.g*., in osteoporosis, can be diagnosed using standard tests, for example, radiographic techniques such as bone densitometry. Osteoporosis is diagnosed, *e.g*., when the bone mineral density is less than or equal to 2.5 standard deviations below that of a young adult reference population. This ratio is reported as a T-score. The World Health Organization has established the following diagnostic guidelines:
T-score -1.0 or greater is "normal"
T-score between -1.0 and -2.5 is "low bone mass" (or "osteopenia")
T-score -2.5 or below is osteoporosis. A GM-CSF antagonist, *e.g*., an antibody can be administered to a patient having low bone mass, *i.e*., osteopenia or to a patient that meets these criteria for osteoporosis.

A GM-CSF antagonist, *e.g*., a GM-CSF antibody, can be administered to patient diagnosed with osteopenia or osteoporosis, or another disorder involving bone loss. A patient that receives the antagonist may also have been diagnosed with other disease, *e.g*., an autoimmune disease such as rheumatoid arthritis. The antagonist, *e.g*., a GM-CSF antibody, can be administered to a patient that has not been diagnosed with rheumatoid arthritis; or to a patient that has not been diagnosed with an autoimmune disease.

Patient response to GM-CSF antagonist treatment can be evaluated by monitoring bone density. A patient that exhibits a therapeutic response to treatment exhibits a slowing of bone density loss in comparison to typical rates of bone loss present in control untreated patients.

### III. GM-CSF Antagonists

As noted above, described herein are methods for treating bone density loss, *e.g*., osteoporosis, by administering a GM-CSF antagonist to a patient. GM-CSF antagonists selectively interfere with the induction of signaling by the GM-CSF receptor, *e.g*., by causing a reduction in the binding of GM-CSF to the receptor. Such antagonists may include antibodies that bind the GM-CSF receptor, antibodies that bind GM-CSF, and other proteins or small molecules that compete for binding of GM-CSF to its receptor or inhibit signaling that normally results from the binding of the ligand to the receptor.

The GM-CSF antagonist used may be a protein, *e.g*., an anti-GM-CSF antibody, an anti-GM-CSF receptor antibody, a soluble GM-CSF receptor, or a modified GM-CSF polypeptide that competes for binding with GM-CSF to a receptor, but is inactive. Such proteins are often produced using recombinant expression technology. Such methods are widely are widely known in the art. General molecular biology methods, including expression methods, can be found, *e.g*., in instruction manuals, such as, Sambrook and Russell (2001) Molecular Cloning: A laboratory manual 3rd ed. Cold Spring Harbor Laboratory Press; Current Protocols in Molecular Biology (2006) John Wiley and Sons ISBN: 0-471-50338-X.

A variety of prokaryotic and/or eukaryotic based protein expression systems may be employed to produce a GM-CSF antagonist protein. Many such systems are widely available from commercial suppliers. These include both prokaryotic and eukaryotic expression systems.

### GM-CSF Antibodies

The antibodies of the invention can be raised against GM-CSF proteins, or fragments, or produced recombinantly.

Methods of preparing polyclonal antibodies are known to the skilled artisan (e.g., Harlow & Lane, Antibodies, A Laboratory manual (1988); Methods in Immunology). Polyclonal antibodies can be raised in a mammal by one or more injections of an immunizing agent and, if desired, an adjuvant. The immunizing agent includes a GM-CSF or fragment thereof.

In some embodiment, a GM-CSF antibody of the invention is purified from human plasma. In such embodiments, the GM-CSF antibody is typically a polyclonal antibody that is isolated from other antibodies present in human plasma. Such an isolation procedure can be performed, *e.g*., using known techniques, such as affinity chromatography.

In some embodiments, the GM-CSF antibody of the invention is a monoclonal antibody. Monoclonal antibodies may be prepared using hybridoma methods, such as those described by Kohler & Milstein, Nature 256:495 (1975). In a hybridoma method, a mouse, hamster, or other appropriate host animal, is typically immunized with an immunizing agent, such as human GM-CSF, to elicit lymphocytes that produce or are capable of producing antibodies that will specifically bind to the immunizing agent. Alternatively, the lymphocytes may be immunized *in vitro.* The immunizing agent preferably includes human GM-CSF protein, fragments thereof, or fusion protein thereof.

Human monoclonal antibodies can be produced using various techniques known in the art, including phage display libraries (Hoogenboom & Winter, J. Mol. Biol. 227:381 (1991); Marks et al., J. Mol. Biol. 222:581 (1991)). The techniques of Cole *et al.* and Boerner *et al.* are also available for the preparation of human monoclonal antibodies (Cole et al., Monoclonal Antibodies and Cancer Therapy, p. 77 (1985) and Boerner et al., J. Immunol. 147(1):86-95 (1991)). Similarly, human antibodies can be made by introducing of human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, *e.g*., in U.S. Patent Nos. 5,545,807; 5,545,806; 5,569,825; 5,625,126; 5,633,425; 5,661,016, and in the following scientific publications: Marks et al., Bio/Technology 10:779-783 (1992); Lonberg et al., Nature 368:856-859 (1994); Morrison, Nature 368:812-13(1994); Fishwild et al., Nature Biotechnology 14:845-51 (1996); Neuberger, Nature Biotechnology 14:826 (1996); Lonberg & Huszar, Intern. Rev. Immunol. 13:65-93 (1995).

In some embodiments the anti-GM-CSF antibodies are chimeric or humanized monoclonal antibodies. As noted *supra,* humanized forms of antibodies are chimeric immunoglobulins in which residues from a complementary determining region (CDR) of human antibody are replaced by residues from a CDR of a non-human species such as mouse, rat or rabbit having the desired specificity, affinity and capacity.

An antibody that is employed in the invention can be in any format. For example, in some embodiments, the antibody can be a complete antibody including a constant region, *e.g.,* a human constant region, or can be a fragment or derivative of a complete antibody, *e.g*., an Fd, a Fab, Fab', F(ab')₂, a scFv, an Fv fragment, or a single domain antibody, such as a nanobody or a camelid antibody. Such antibodies may additionally be recombinantly engineered by methods well known to persons of skill in the art. As noted above, such antibodies can be produced using known techniques.

In some embodiments of the invention, the antibody is additionally engineered to reduced immunogenicity, *e.g*., so that the antibody is suitable for repeat administration. Methods for generating antibodies with reduced immunogenicity include humanization/humaneering procedures and modification techniques such as de-immunization, in which an antibody is further engineered, *e.g*., in one or more framework regions, to remove T cell epitopes.

In some embodiments, the antibody is a humaneered antibody. A humaneered antibody is an engineered human antibody having a binding specificity of a reference antibody, obtained by joining a DNA sequence encoding a binding specificity determinant (BSD) from a CDR, *e.g*., a CDR3 region, of the heavy chain of the reference antibody to human V_{H} segment sequence and a light chain CDR, *e.g*. a CDR3 region, BSD from the reference antibody to a human V_{L} segment sequence. Methods for humaneering are provided in US patent application publication no. 20050255552 and US patent application publication no. 20060134098.

An antibody can further be de-immunized to remove one or more predicted T-cell epitopes from the V-region of an antibody. Such procedures are described, for example, in WO 00/34317.

In some embodiments, the variable region is comprised of human V-gene sequences. For example, a variable region sequence can have at least 80% identity, or at least 85% identity, at least 90% identity, at least 95% identity, at least 96% identity, at least 97% identity, at least 98% identity, or at least 99% identity, or greater, with a human germline V-gene sequence.

An antibody used in the invention can include a human constant region. The constant region of the light chain may be a human kappa or lambda constant region. The heavy chain constant region is often a gamma chain constant region, for example, a gamma-1, gamma-2, gamma-3, or gamma-4 constant region.

In some embodiments, *e.g*., where the antibody is a fragment, the antibody can be conjugated to another molecule, *e.g*., to provide an extended half-life *in vivo* such as a polyethylene glycol (pegylation) or serum albumin. Examples of PEGylation of antibody fragments are provided in Knight et al (2004) Platelets 15: 409 (for abciximab); Pedley et al (1994) Br. J. Cancer 70: 1126 (for an anti-CEA antibody) Chapman et al (1999) Nature Biotech. 17 : 780.

### Antibody Specificity

An antibody for use in the invention binds to GM-CSF. Any number of techniques can be used to determine antibody binding specificity. *See, e.g.,* Harlow & Lane, Antibodies, A Laboratory Manual (1988) for a description of immunoassay formats and conditions that can be used to determine specific immunoreactivity of an antibody.

An exemplary antibody suitable for use with the present invention is c19/2. In some embodiments, a monoclonal antibody that competes for binding to the same epitope as c19/2, or that binds the same epitope as c19/2, is used. The ability of a particular antibody to recognize the same epitope as another antibody is typically determined by the ability of the first antibody to competitively inhibit binding of the second antibody to the antigen. Any of a number of competitive binding assays can be used to measure competition between two antibodies to the same antigen. For example, a sandwich ELISA assay can be used for this purpose. This is carried out by using a capture antibody to coat the surface of a well. A subsaturating concentration of tagged-antigen is then added to the capture surface. This protein will be bound to the antibody through a specific antibody:epitope interaction. After washing a second antibody, which has been covalently linked to a detectable moiety (e.g., HRP, with the labeled antibody being defined as the detection antibody) is added to the ELISA. If this antibody recognizes the same epitope as the capture antibody it will be unable to bind to the target protein as that particular epitope will no longer be available for binding. If however this second antibody recognizes a different epitope on the target protein it will be able to bind and this binding can be detected by quantifying the level of activity (and hence antibody bound) using a relevant substrate. The background is defined by using a single antibody as both capture and detection antibody, whereas the maximal signal can be established by capturing with an antigen specific antibody and detecting with an antibody to the tag on the antigen. By using the background and maximal signals as references, antibodies can be assessed in a pair-wise manner to determine epitope specificity.

A first antibody is considered to competitively inhibit binding of a second antibody, if binding of the second antibody to the antigen is reduced by at least 30%, usually at least about 40%, 50%, 60% or 75%, and often by at least about 90%, in the presence of the first antibody using any of the assays described above.

### Epitope Mapping

In some embodiments of the invention, an antibody is employed that binds to the same epitope as a known antibody, *e.g*., c19/2. Method of mapping epitopes are well known in the art. For example, one approach to the localization of functionally active regions of human granulocyte-macrophage colony-stimulating factor (hGM-CSF) is to map the epitopes recognized by neutralizing anti-hGM-CSF monoclonal antibodies. For example, the epitope to which c19/2 (which has the same variable regions as the neutralizing antibody LMM102) binds has been defined using proteolytic fragments obtained by enzymatic digestion of bacterially synthesized hGM-CSF (Dempsey, et al., Hybridoma 9:545-558, 1990). RP-HPLC fractionation of a tryptic digest resulted in the identification of an immunoreactive "tryptic core" peptide containing 66 amino acids (52% of the protein). Further digestion of this "tryptic core" with S. aureus V8 protease produced a unique immunoreactive hGM-CSF product comprising two peptides, residues 86-93 and 112-127, linked by a disulfide bond between residues 88 and 121. The individual peptides, were not recognized by the antibody.

### Determining Binding Affinity

The antibodies described herein may have a high affinity binding for human GM-CSF. High affinity binding between an antibody and an antigen exists if the dissociation constant (K_{D}) of the antibody is < 100 pM. A variety of methods can be used to determine the binding affinity of an antibody for its target antigen such as surface plasmon resonance assays, saturation assays, or immunoassays such as ELISA or RIA, as are well known to persons of skill in the art. An exemplary method for determining binding affinity is by surface plasmon resonance analysis on a BIAcore™ 2000 instrument (Biacore AB, Freiburg, Germany) using CM5 sensor chips, as described by Krinner et al., (2007) Mol. Immunol. Feb;44(5):916-25. (Epub 2006 May 11)).

### Cell Proliferation Assay for Identifying Neutralizing Antibodies

Neutralizing antibodies may be identified using any number of assays that assess GM-CSF function. For example, cell-based assays for GM-CSF receptor signaling, such as assays which determine the rate of proliferation of a GM-CSF-dependent cell line in response to a limiting amount of GM-CSF, are conveniently used. The human TF-1 cell line is suitable for use in such an assay. See, Krinner et al., (2007) Mol. Immunol. In some embodiments, the neutralizing antibodies of the invention inhibit GM-CSF-stimulated TF-1 cell proliferation by at least 50% when a GM-CSF concentration is used which stimulates 90% maximal TF-1 cell proliferation. In other embodiments, the neutralizing antibodies inhibit GM-CSF stimulated proliferation by at least 90%. Additional assays suitable for use in identifying neutralizing antibodies suitable for use with the present invention will be well known to persons of skill in the art.

### Exemplary Antibodies

Antibodies for use in the invention are known in the art and can be produced using routine techniques. Exemplary antibodies are described. It is understood that the exemplary antibodies can be engineered in accordance with the procedures known in the art and summarized herein to produce antibody fragments, chimeras, and the like by either chemical or recombinant technology.

An exemplary chimeric antibody suitable for use as a GM-CSF antagonist is c19/2. The c/19/2 antibody binds GM-CSF with a monovalent binding affinity of about 10pM as determined by surface plasmon resonance analysis. SEQ ID NOs 1 and 2 show the heavy and light chain variable region sequence of c19/2 (*e.g*., WO03/068920). The CDRs, as defined according to Kabat, are:

| | |
|---|---|
| CDRH1 | DYNIH |
| CDRH2 | YIAPYSGGTGYNQEFKN |
| CDRH3 | RDRFPYYFDY |
| CDRL1 | KASQNVGSNVA |
| CDRL2 | SASYRSG |
| CDRL3 | QQFNRSPLT. |

The CDRs can also be determined using other well known definitions in the art, *e.g*., Chothia, international ImMunoGeneTics database (IMGT), and AbM.

The GM-CSF epitope recognized by c19/2 has been identified as a product that has two peptides, residues 86-93 and residues 112-127, linked by a disulfide bond between residues 88 and 121. The c19/2 antibody inhibits the GM-CSF-dependent proliferation of a human TF-1 leukemia cell line with an EC₅₀ of 30 pM when the cells are stimulated with 0.5 ng/ml GM-CSF.

An antibody for administration, such as c19/2, can be additionally humaneered. For example, the c19/2 antibody can be further engineered to contain human V gene segments.

Another exemplary neutralizing anti-GM-CSF antibody is the E10 antibody described in Li et al., (2006) PNAS 103(10):3557-3562. E10 is an IgG class antibody that has an 870 pM binding affinity for GM-CSF. The antibody is specific for binding to human GM-CSF as shown in an ELISA assay, and shows strong neutralizing activity as assessed with a TF1 cell proliferation assay.

An additional exemplary neutralizing anti-GM-CSF antibody is the MT203 antibody described by Krinner et al., (Mol Immunol. 44:916-25, 2007; Epub 2006 May 112006). MT203 is an IgG1 class antibody that binds GM-CSF with picomolar affinity. The antibody shows potent inhibitory activity as assessed by TF-1 cell proliferation assay and its ability to block IL-8 production in U937 cells. Additional GM-CSF antibodies are described, *e.g*., by Steidl et al. in WO2006122797.

Additional antibodies suitable for use with the present invention will be known to persons of skill in the art.

### Non-Antibody GM-CSF Antagonists

Other proteins which may interfere with the productive interaction of GM-CSF with its receptor include mutant GM-CSF proteins and secreted proteins comprising at least part of the extracellular portion of one or both of the GM-CSF receptor chains that bind to GM-CSF and compete with binding to cell-surface receptor. For example, a soluble GM-CSFR antagonist can be prepared by fusing the coding region of the sGM-CSFRalpha with the CH2-CH3 regions of murine IgG2a. An exemplary soluble GM-CSF receptor is described by Raines et al. (1991) Proc. Natl. Acad. Sci USA 88: 8203. Examples of GM-CSFRalpha-Fc fusion proteins are provided, *e.g*., in Brown et al., Blood 85:1488, 1995; Monfardini et al., J. Biol. Chem 273:7657-7667, 1998; and Sayani et al., Blood 95:461-469, 2000. The Fc component of such a fusion can be engineered to modulate binding, *e.g*., to increase binding, to the Fc receptor.

Other GM-CSF antagonist include GM-CSF mutants. For example, GM-CSF having a mutation of amino acid residue 21 of GM-CSF to Arginine or Lysine (E21R or E221K) described by Hercus et al., Proc. Natl. Acad. Sci USA 91:5838, 1994 has been shown to have *in vivo* activity in preventing dissemination of GM-CSF-dependent leukemia cells in mouse xenograft models (Iversen et al. Blood 90:4910, 1997). As appreciated by one of skill in the art, such antagonists can include conservatively modified variants of GM-CSF that have substitutions, such as the substitution noted at amino acid residue 21, or GM-CSF variants that have, *e.g*., amino acid analogs to prolong half-life.

Other GM-CSF peptide inhibitors are also known, *e.g.,* cyclic peptides, *e.g.,* Monfardini, et al., J. Biol. Chem. 271: 1966-1971, 1996.

GM-CSF antagonists include an "antibody mimetic" that targets and binds to the antigen in a manner similar to antibodies. Certain of these "antibody mimics" use non-immunoglobulin protein scaffolds as alternative protein frameworks for the variable regions of antibodies. For example, Ku et al. (Proc. Natl. Acad. Sci. U.S.A. 92(14):6552-6556 (1995)) discloses an alternative to antibodies based on cytochrome b562 in which two of the loops of cytochrome b562 were randomized and selected for binding against bovine serum albumin. The individual mutants were found to bind selectively with BSA similarly with anti-BSA antibodies.

U.S. Patent Nos. 6,818,418 and 7,115,396 disclose an antibody mimic featuring a fibronectin or fibronectin-like protein scaffold and at least one variable loop. Known as Adnectins, these fibronectin-based antibody mimics exhibit many of the same characteristics of natural or engineered antibodies, including high affinity and specificity for any targeted ligand. The structure of these fibronectin-based antibody mimics is similar to the structure of the variable region of the IgG heavy chain. Therefore, these mimics display antigen binding properties similar in nature and affinity to those of native antibodies. Further, these fibronectin-based antibody mimics exhibit certain benefits over antibodies and antibody fragments. For example, these antibody mimics do not rely on disulfide bonds for native fold stability, and are, therefore, stable under conditions which would normally break down antibodies. In addition, since the structure of these fibronectin-based antibody mimics is similar, to that of the IgG heavy chain, the process for loop randomization and shuffling may be employed in vitro that is similar to the process of affinity maturation of antibodies *in vivo*.

Beste et al. (Proc. Natl. Acad. Sci. U.S.A. 96(5):1898-1903 (1999)) disclose an antibody mimic based on a lipocalin scaffold (Anticalin®). Lipocalins are composed of a β-barrel with four hypervariable loops at the terminus of the protein. The loops were subjected to random mutagenesis and selected for binding with, for example, fluorescein. Three variants exhibited specific binding with fluorescein, with one variant showing binding similar to that of an anti-fluorescein antibody. Further analysis revealed that all of the randomized positions are variable, indicating that Anticalin® would be suitable to be used as an alternative to antibodies. Thus, Anticalins® are small, single chain peptides, typically between 160 and 180 residues, which provides several advantages over antibodies, including decreased cost of production, increased stability in storage and decreased immunological reaction.

U.S. Patent No. 5,770,380 discloses a synthetic antibody mimetic using the rigid, non-peptide organic scaffold of calixarene, attached with multiple variable peptide loops used as binding sites. The peptide loops all project from the same side geometrically from the calixarene, with respect to each other. Because of this geometric confirmation, all of the loops are available for binding, increasing the binding affinity to a ligand. However, in comparison to other antibody mimics, the calixarene-based antibody mimic does not consist exclusively of a peptide, and therefore it is less vulnerable to attack by protease enzymes. Neither does the scaffold consist purely of a peptide, DNA or RNA, meaning this antibody mimic is relatively stable in extreme environmental conditions and has a long life span. Further, since the calixarene-based antibody mimic is relatively small, it is less likely to produce an immunogenic response.

Murali et al. (Cell Mol. Biol 49(2):209-216 (2003)) describe a methodology for reducing antibodies into smaller peptidomimetics, they term "antibody like binding peptidomimetics" (ABiP) which may also be useful as an alternative to antibodies.

In addition to non-immunoglobulin protein frameworks, antibody properties have also been mimicked in compounds comprising RNA molecules and unnatural oligomers (e.g., protease inhibitors, benzodiazepines, purine derivatives and beta-turn mimics). Accordingly, non-antibody GM-CSF antagonists can also include such compounds.

### IV. Therapeutic Administration

The methods described herein comprise administering a GM-CSF antagonist, (e.g., an anti-GM-CSF antibody) as a pharmaceutical composition to a patient having bone density loss, *e.g.*, a patient with osteoporosis, in a therapeutically effective amount using a dosing regimen suitable for treatment of the disease. The composition can be formulated for use in a variety of drug delivery systems. One or more physiologically acceptable excipients or carriers can also be included in the compositions for proper formulation. Suitable formulations for use in the present invention are found in Remington's Pharmaceutical Sciences, Mack Publishing Company, Philadelphia, PA, 17th ed. (1985). For a brief review of methods for drug delivery, see, Langer, Science 249: 1527-1533 (1990).

The GM-CSF antagonist for use in these methods is provided in a solution suitable for injection into the patient such as a sterile isotonic aqueous solution for injection. The GM-CSF antagonist is dissolved or suspended at a suitable concentration in an acceptable carrier. The carrier may be aqueous, e.g., water, saline, phosphate buffered saline, and the like. The compositions may contain auxiliary pharmaceutical substances as required to approximate physiological conditions, such as pH adjusting and buffering agents, tonicity adjusting agents, and the like.

GM-CSF antagonist pharmaceutical compositions may be administered to a patient with bone loss in an amount sufficient to at least partially arrest bone loss and/or symptoms of bone loss and its complications. An amount adequate to accomplish this is defined as a "therapeutically effective dose." A therapeutically effective dose is determined by monitoring a patient's response to therapy. A typical benchmark is a reduction in bone loss or the rate of bone loss, *e.g*., compared to patients who are not treated for osteopenia. Amounts effective for this use will depend upon the severity of the disease and the general state of the patient's health, including other factors such as age, weight, gender, administration route, etc. The GM-CSF antagonist is preferably administered in an amount that does not induce neutropenia when administered either alone or in combination with another therapeutic agent. Single or multiple administrations of the antagonist may be administered depending on the dosage and frequency as required and tolerated by the patient. In any event, the methods provide a sufficient quantity of GM-CSF antagonist to effectively treat the patient.

The GM-CSF antagonist used to treat a patient exhibiting loss of bone density may be provided in combination therapy with another agent, such as a bisphosphonate or bone-enhancing minerals such as fluoride or calcium; calcitonin, hormone replacement therapy, or other therapeutic agents used to treat bone loss. Other therapeutic agents include RANKL antagonists such as osteoprotegerin (OPG), which is a natural RANKL antagonist, *e.g*., OPG-Fc fusion proteins, and antibodies to RANKL, *e.g*., denosumab.

A patient may undergo treatment with the GM-CSF antagonist and one or more additional another therapeutic agents either concomitantly or sequentially. A patient may initially be treated with an agent, *e.g*., a bisphosphonate, and then receive treatment with the GM-CSF antagonist after treatment with the bisphosphonate has been discontinued. A lower dose, and/or less frequent dosages, of the additional therapeutic agents, *e.g*., a bisphosphonate, may be used when the patient also undergoes treatment with a GM-CSF antagonist, *e.g*., a GM-CSF antibody, in comparison to the amount of therapeutic agent, *e.g*., a bisphosphonate, typically administered to a patient. As understood in the art, the dosages and frequency of administration of the GM-CSF antagonist may also be adjusted when used in combination with another therapeutic agent for bone loss.

### A. Administration

An GM-CSF antibody may be administered by injection or infusion through any suitable route including but not limited to intravenous, sub-cutaneous, intramuscular or intraperitoneal routes. The GM-CSF antibody may be administered directly to a site of localized bone loss, *e.g*., to the mandible or gum area where periodontal disease leads to site-specific bone loss.

The antibody may be stored at 10 mg/ml in sterile isotonic aqueous saline solution for injection at 4°C and is diluted in either 100 ml or 200 ml 0.9% sodium chloride for injection prior to administration to the patient. The antibody may be administered by intravenous infusion over the course of 1 hour at a dose of between 0.2 and 10 mg/kg. The antibody may be administered by intravenous infusion over a period of between 15 minutes and 2 hours. The administration procedure may be via sub-cutaneous bolus injection.

### B. Dosing

The dose of antagonist is chosen in order to provide effective therapy for the patient and is in the range of less than 0.1 mg/kg body weight to 25 mg/kg body weight or in the range 1 mg - 2 g per patient. Preferably the dose is in the range 1 - 10 mg/kg or approximately 50 mg - 1000 mg / patient. The dose may be repeated at an appropriate frequency which may be in the range once per day to once every three months, depending on the pharmacokinetics of the antagonists (e.g. half-life of the antibody in the circulation) and the pharmacodynamic response (e.g. the duration of the therapeutic effect of the antibody). Where the antagonist is an antibody or modified antibody fragment, the *in vivo* half-life of between about 7 and about 25 days and antibody dosing is repeated between once per week and once every 3 months. In other embodiments, the antibody is administered approximately once per month.

### EXAMPLES

### Example 1. A GM-CSF antibodies decreases osteopenia in an ovariectomized mouse model

The effects of a mouse anti-GM-CSF antibody that neutralizes GM-CSF on osteoporosis was tested in a mouse model. Experimental osteoporosis was induced by ovariectomy in C3H mice. Four groups of eight mice each were evaluated early in the course of clinical disease. Figure 1 shows the four groups. Group 3 corresponds to the group that received anti-GM-CSF antibody. In Groups 2 and 3, treatment was 3x weekly with 0.3 mg anti-GM-CSF or 0.1 mg/kg alendronate for 8 weeks beginning 4 days post-surgery.

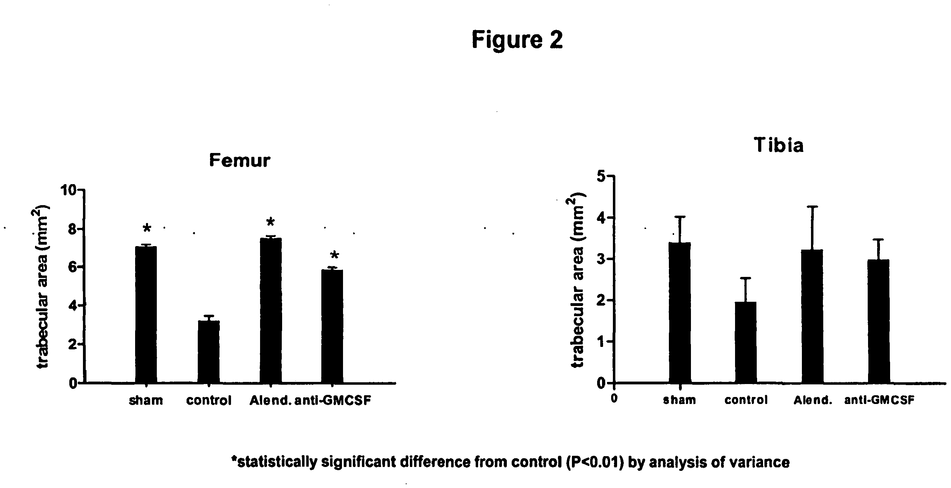

Figure 2 shows the results of histomorphometric analysis of canellous bone density. Animals in Group 3 (treated with anti-GM-CSF antibody) showed greater bone density relative to Group I control ovariectomized animals that received phosphate buffered saline.

Figure 3 shows photomicrographs of section of epiphyseal area of decalcified tibia. Panels 1-4 depict tibia from groups 1-4, respectively. Group 3 animals demonstrated increased in size, number, and density of trabeculae relative to Group 1 animals.

Figure 4 shows the osteoblast and osteoclast morphology and activity for the group groups. In Group 4, in which the animals were subjected to sham surgery, the osteoclast numbers and activity were normal. Group 3 animals that were treated with GM-CSF antibody appeared to have significantly fewer osteoclasts that histologically showed inactive morphology. In Group 2, osteoclasts were abundant in numbers and appeared highly active. In Group 1, the number of osteoclasts appeared to be reduced and appeared to be slightly less active.

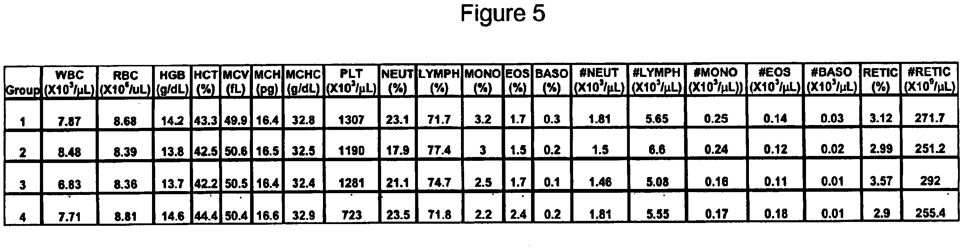

Hematological measurements, clinical chemistry, organ weights, organ weights normalized to body weight, and organ weights normalized to brain weight were not substantially different in the treatment groups. Figure 5 shows hematological data. Of note, administration of GM-CSF antibody did not result in a decrease in neutrophil numbers.

### Example 2 - Exemplary humaneered antibodies to GM-CSF

A panel of humaneered Fab' molecules with the specificity of c19/2 were generated from epitope-focused human V-segment libraries as described in US patent application 20060134098.

Fab' fragments were expressed from E. coli. Cells were grown in 2xYT medium to an OD600 of 0.6. Expression was induced using IPTG for 3 hours at 33°C. Assembled Fab' was obtained from periplasmic fractions and purified by affinity chromatography using Streptococcal Protein G (HiTrap Protein G HP columns; GE Healthcare) according to standard methods. Fab's were eluted in pH 2.0 buffer, immediately adjusted to pH 7.0 and dialyzed against PBS pH7.4.

Binding kinetics were analyzed by Biacore 3000 surface plasmon resonance (SPR). Recombinant human GM-CSF antigen was biotinylated and immobilized on a streptavidin CM5 sensor chip. Fab samples were diluted to a starting concentration of 3 nM and run in a 3 fold dilution series. Assays were run in 10 mM HEPES, 150 mM NaCl, 0.1 mg/mL BSA and 0.005% p20 at pH 7.4 and 37°C. Each concentration was tested twice. Fab' binding assays were run on two antigen density surfaces providing duplicate data sets. The mean affinity (K_{D}) for each of 6 humaneered anti-GM-CSF Fab clones, calculated using a 1:1 Langmuir binding model, is shown in Table 1.

Fabs were tested for GM-CSF neutralization using a TF-1 cell proliferation assay. GM-CSF-dependent proliferation of human TF-1 cells was measured after incubation for 4 days with 0.5 ng/ml GM-CSF using a MTS assay (Cell titer 96, Promega) to determine viable cells. All Fabs inhibited cell proliferation in this assay indicating that these are neutralizing antibodies. There is a good correlation between relative affinities of the anti-GM-CSF Fabs and EC₅₀ in the cell-based assay. Anti-GM-CSF antibodies with monovalent affinities in the range 18 pM - 104 pM demonstrate effective neutralization of GM-CSF in the cell-based assay.

**Table 1: Affinity of anti-GM-CSF Fabs determined by surface plasmon resonance analysis in comparison with activity (EC₅₀) in a GM-CSF dependent TF-1 cell proliferation assay**

| **Fab** | **Monovalent binding affinity determined by SPR (pM)** | **EC₅₀(pM) in TF-1 cell proliferation assay** |
|---|---|---|
| 94 | 18 | 165 |
| 104 | 19 | 239 |
| 77 | 29 | 404 |
| 92 | 58 | 539 |
| 42 | 104 | 3200 |
| 44 | 81 | 7000 |

### Example 3 - Clinical protocol for delivery of anti-GM-CSF antibody

An anti-GM-CSF antibody is stored at 10 mg/ml in sterile isotonic aqueous saline solution for injection at 4°C and is diluted in either 100 ml or 200 ml 0.9% sodium chloride for injection prior to administration to the patient. The antibody is administered to a patient having osteoporosis by intravenous infusion over the course of 1 hour at a dose of between 0.2 and 10 mg/kg.

### Exemplary sequences

SEQ ID NO 1: amino acid sequence for murine 19/2 heavy chain variable region

Met Glu Leu Ile Met Leu Phe Leu Leu Ser Gly Thr Ala Gly Val His

Ser Glu Val Gln Leu Gln Gln Ser Gly Pro Glu Leu Val Lys Pro Gly

Ala Ser Val Lys Ile Ser Cys Lys Ala Ser Gly Tyr Thr Phe Thr Asp

Tyr Asn Ile His Trp Val Lys Gln Ser His Gly Lys Ser Leu Asp Trp

Ile Gly Tyr Ile Ala Pro Tyr Ser Gly Gly Thr Gly Tyr Asn Gln Glu

Phe Lys Asn Arg Ala Thr Leu Thr Val Asp Lys Ser Ser Ser Thr Ala

Tyr Met Glu Leu Arg Ser Leu Thr Ser Asp Asp Ser Ala Val Tyr Tyr

Cys Ala Arg Arg Asp Arg Phe Pro Tyr Tyr Phe Asp Tyr Trp Gly Gln Gly

Thr Thr Leu Arg Val Ser Ser Val Ser Gly Ser

SEQ ID NO 2: amino acid sequence for murine 19/2 light chain variable region

Met Gly Phe Lys Met Glu Ser Gln Ile Gln Val Phe Val Tyr Met Leu

Leu Trp Leu Ser Gly Val Asp Gly Asp Ile Val Met Ile Gln Ser Gln

Lys Phe Val Ser Thr Ser Val Gly Asp Arg Val Asn Ile Thr Cys Lys

Ala Ser Gln Asn Val Gly Ser Asn Val Ala Trp Leu Gln Gln Lys Pro

Gly Gln Ser Pro Lys Thr Leu Ile Tyr Ser Ala Ser Tyr Arg Ser Gly

Arg Val Pro Asp Arg Phe Thr Gly Ser Gly Ser Gly Thr Asp Phe Ile

Leu Thr Ile Thr Thr Val Gln Ser Glu Asp Leu Ala Glu Tyr Phe Cys

Gln Gln Phe Asn Arg Ser Pro Leu Thr Phe Gly Ser Gly Thr Lys Leu

Glu Leu Lys Arg Ala Asp Ala Ala Pro Thr Val Ser Ile Phe Pro Pro

Ser Ser Lys Gly Glu Phe

## Claims

1. A neutralizing anti-GM-CSF antibody for use in a method for treating a patient that has osteopenia that is a general loss of bone density below normal that is not site-specific, the method comprising administering a therapeutically effective amount of said antibody to the patient in an amount sufficient to reduce the symptoms of bone loss, wherein said antibody binds to GM-CSF with a dissociation constant (K_{D}) less than about 100 pM.

2. The neutralizing anti-GM-CSF antibody according to claim 1 for the use of claim 1, wherein the patient has osteoporosis.

3. The neutralizing anti-GM-CSF antibody according to claim 1 or 2 for the use of claim 1 or 2, wherein the patient has not been diagnosed as having rheumatoid arthritis.

4. The neutralizing anti-GM-CSF antibody according to claims 1 to 3 for the use of any one of claims 1 to 3, wherein the antibody is a monoclonal antibody.

5. The neutralizing anti-GM-CSF antibody according to claims 1 to 4 for the use of any one of claims 1 to 4, wherein the antibody:
(a) binds to GM-CSF with a dissociation constant (K_{D}) ranging from about 5 pM to about 50 pM; and/or
(b) is a recombinant or chimeric antibody; and/or
(c) comprises a human variable region or is a human antibody.

6. The neutralizing anti-GM-CSF antibody according to claims 1 to 5 for the use of any one of claims 1 to 5, wherein the antibody:
(a) is an antibody fragment that is a Fab, a Fab', a F(ab')₂, a scFv, or a dAB, optionally wherein the antibody fragment is conjugated to polyethylene glycol; or
(b) comprises a human light chain constant region and/or a human heavy chain constant region, optionally wherein the human heavy chain constant region is a gamma chain.

7. The neutralizing anti-GM-CSF antibody according to claims 1 to 6 for the use of any one of claims 1 to 6, wherein the antibody binds to the same epitope as a chimeric 19/2 having a V_{H} region amino acid sequence set forth in SEQ ID NO: 1 and a V_{L} region amino acid sequence set forth in SEQ ID NO: 2.

8. The neutralizing anti-GM-CSF antibody according to claims 1 to 6 for the use of any one of claims 1 to 6, wherein the antibody comprises the V_{H} and V_{L} regions of a chimeric 19/2 having a V_{H} region amino acid sequence set forth in SEQ ID NO: 1 and a V_{L} region amino acid sequence set forth in SEQ ID NO: 2.

9. The neutralizing anti-GM-CSF antibody according to claim 8 for the use of claim 8, wherein the antibody comprises a human heavy chain constant region.

10. The neutralizing anti-GM-CSF antibody according to claim 9 for the use of claim 9, wherein the human heavy chain constant region is a gamma region.

11. The neutralizing anti-GM-CSF antibody according to claims 1 to 6 for the use of any one of claims 1 to 6, wherein:
(a) the antibody comprises the V_{H} region and V_{L} region CDR1, CDR2, and CDR3 of a chimeric 19/2 having a V_{H} region amino acid sequence set forth in SEQ ID NO: 1 and a V_{L} region amino acid sequence set forth in SEQ ID NO: 2; or
(b) the antibody comprises the V_{H} region CDR3 and V_{L} region CDR3 of a chimeric 19/2 having a V_{H} region amino acid sequence set forth in SEQ ID NO: 1 and a V_{L} region amino acid sequence set forth in SEQ ID NO: 2.

12. The neutralizing anti-GM-CSF antibody according to any one of the preceding claims for use of any one of the preceding claims, wherein the method further comprises administering a therapeutic agent selected from the group consisting of a bisphosphonate, raloxifene, teriparatide, strontium ranelate, a RANKL antagonist, and an osteoprotegerin (OPG)-Fc fusion protein.

13. The neutralizing anti-GM-CSF antibody according to claim 12 for the use of claim 12, wherein the bisphosphonate is selected from the group consisting of alendronate, etidronate, risedronate and ibandronic acid.

14. The neutralizing anti-GM-CSF antibody according to claim 1 for the use of claim 1, wherein said antibody (a) comprises a humaneered Fab' with the binding specificity of a chimeric 19/2 having a V_{H} region amino acid sequence set forth in SEQ ID NO: 1 and a V_{L} region amino acid sequence set forth in SEQ ID NO: 2 and (b) binds to GM-CSF with a dissociation constant (K_{D}) ranging from about 5 to about 50 pM.

15. The neutralizing anti-GM-CSF antibody according to any one of the preceding claims for the use of any one of the preceding claims, wherein the osteopenia results from estrogen deficiency.

## Patentansprüche

1. Neutralisierender Anti-GM-CSF-Antikörper zur Verwendung in einem Verfahren zur Behandlung eines Patienten, der an Osteopenie leidet, was einen allgemeinen Verlust der Knochendichte unter normal bedeutet, der nicht ortsspezifisch ist, wobei das Verfahren das Verabreichen einer therapeutisch wirksamen Menge des Antikörpers an den Patienten umfasst, in einer Menge, die ausreicht, um die Symptome des Knochenverlustes zu reduzieren, wobei der Antikörper an GM-CSF mit einer Dissoziationskonstante (K_{D}) von kleiner als 100 pM bindet.

2. Neutralisierender Anti-GM-CSF-Antikörper gemäß Anspruch 1 zur Verwendung gemäß Anspruch 1, wobei der Patient Osteoporose hat.

3. Neutralisierender Anti-GM-CSF-Antikörper gemäß Anspruch 1 oder 2 zur Verwendung gemäß Anspruch 1 oder 2, wobei bei dem Patienten keine rheumatoide Arthritis diagnostiziert wurde.

4. Neutralisierender Anti-GM-CSF-Antikörper gemäß den Ansprüchen 1 bis 3 zur Verwendung gemäß einem der Ansprüche 1 bis 3, wobei der Antikörper ein monoklonaler Antikörper ist.

5. Neutralisierender Anti-GM-CSF-Antikörper gemäß den Ansprüchen 1 bis 4 zur Verwendung gemäß einem der Ansprüche 1 bis 4, wobei der Antikörper:
(a) an GM-CSF mit einer Dissoziationskonstante (K_{D}) im Bereich von ungefähr 5 pM bis ungefähr 50 pM bindet; und/oder
(b) ein rekombinanter oder chimärer Antikörper ist; und/oder
(c) eine humane variable Region umfasst oder ein humaner Antikörper ist.

6. Neutralisierender Anti-GM-CSF-Antikörper gemäß den Ansprüchen 1 bis 5 zur Verwendung gemäß einem der Ansprüche 1 bis 5, wobei der Antikörper:
(a) ein Antikörperfragment ist, das ein Fab, ein Fab', ein F(ab')₂, ein scFv oder ein dAB ist, gegebenenfalls wobei das Antikörperfragment an Polyethylenglykol konjugiert ist; oder
(b) eine konstante Region einer humanen leichten Kette und/oder eine konstante Region einer humanen schweren Kette umfasst, gegebenenfalls, wobei die konstante Region der humanen schweren Kette eine gamma-Kette ist.

7. Neutralisierender Anti-GM-CSF-Antikörper gemäß den Ansprüchen 1 bis 6 zur Verwendung gemäß einem der Ansprüche 1 bis 6, wobei der Antikörper an dasselbe Epitop bindet wie ein chimärer 19/2, der eine V_{H}-Aminosäuresequenz-Region, dargelegt in SEQ ID NO:1, und eine V_{L}-Aminosäuresequenz-Region, dargelegt in SEQ ID NO:2 hat.

8. Neutralisierender Anti-GM-CSF-Antikörper gemäß den Ansprüchen 1 bis 6 zur Verwendung gemäß einem der Ansprüche 1 bis 6, wobei der Antikörper die V_{H}- und V_{L}-Regionen eines chimären 19/2 umfasst, der eine V_{H}-Aminosäuresequenz-Region, dargelegt in SEQ ID NO:1, und eine V_{L}-Aminosäuresequenz-Region, dargelegt in SEQ ID NO:2 hat.

9. Neutralisierender Anti-GM-CSF-Antikörper gemäß Anspruch 8 zur Verwendung gemäß Anspruch 8, wobei der Antikörper eine konstante Region einer humanen schweren Kette umfasst.

10. Neutralisierender Anti-GM-CSF-Antikörper gemäß Anspruch 9 zur Verwendung gemäß Anspruch 9, wobei die konstante Region der humanen schweren Kette eine gamma-Region ist.

11. Neutralisierender Anti-GM-CSF-Antikörper gemäß den Ansprüchen 1 bis 6 zur Verwendung gemäß einem der Ansprüche 1 bis 6, wobei:
(a) der Antikörper die CDR1, CDR2 und CDR3 der V_{H}-Region und V_{L}-Region eines chimären 19/2 umfasst, der eine Aminosäuresequenz der V_{H}-Region, dargelegt in SEQ ID NO:1, und eine Aminosäuresequenz der V_{L}-Region, dargelegt in SEQ ID NO:2 hat; oder
(b) der Antikörper die CDR3 der V_{H}-Region und CDR3 der V_{L}-Region eines chimären 19/2 umfasst, der eine Aminosäuresequenz der V_{H}-Region, dargelegt in SEQ ID NO:1, und eine Aminosäuresequenz der V_{L}-Region, dargelegt in SEQ ID NO:2 hat.

12. Neutralisierender Anti-GM-CSF-Antikörper gemäß einem der vorhergehenden Ansprüche zur Verwendung gemäß einem der vorhergehenden Ansprüche, wobei das Verfahren weiter das Verabreichen eines therapeutischen Mittels umfasst, ausgewählt aus der Gruppe, bestehend aus einem Bisphosphonat, Raloxifen, Teriparatid, Strontiumranelat, einem RANKL-Antagonisten und einem Osteoprotegerin (OPG)-Fc-Fusionsprotein.

13. Neutralisierender Anti-GM-CSF-Antikörper gemäß Anspruch 12 zur Verwendung gemäß Anspruch 12, wobei das Bisphosphonat ausgewählt ist aus der Gruppe, bestehend aus Alendronat, Etidronat, Risedronat und Ibandronsäure.

14. Neutralisierender Anti-GM-CSF-Antikörper gemäß Anspruch 1 zur Verwendung gemäß Anspruch 1, wobei der Antikörper (a) ein humanisiertes Fab' mit der Bindungsspezifität eines chimären 19/2 umfasst, der eine Aminosäuresequenz der V_{H}-Region, dargelegt in SEQ ID NO:1, und eine Aminosäuresequenz der V_{L}-Region, dargelegt in SEQ ID NO:2 hat, und (b) an GM-CSF mit einer Dissoziationskonstante (K_{D}) im Bereich von ungefähr 5 bis ungefähr 50 pM bindet.

15. Neutralisierender Anti-GM-CSF-Antikörper gemäß einem der vorhergehenden Ansprüche zur Verwendung gemäß einem der vorhergehenden Ansprüche, wobei die Osteopenie aus Östrogenmangel resultiert.

## Revendications

1. Anticorps anti-GM-CSF neutralisant pour utilisation dans un procédé de traitement d'un patient présentant une ostéopénie qui est une perte générale de densité osseuse jusqu'au-dessous de la normale, sans spécificité de site, lequel procédé comporte le fait d'administrer au patient ledit anticorps en une quantité à effet thérapeutique, soit en une quantité suffisante pour faire diminuer les symptômes de perte osseuse, lequel anticorps se lie au facteur GM-CSF avec une constante de dissociation K_{D} inférieure à environ 100 pM.

2. Anticorps anti-GM-CSF neutralisant conforme à la revendication 1 pour l'utilisation de la revendication 1 chez un patient présentant une ostéoporose.

3. Anticorps anti-GM-CSF neutralisant conforme à la revendication 1 ou 2 pour l'utilisation de la revendication 1 ou 2 chez un patient chez lequel on n'a pas diagnostiqué de polyarthrite rhumatoïde.

4. Anticorps anti-GM-CSF neutralisant conforme à l'une des revendications 1 à 3 pour l'utilisation de l'une des revendications 1 à 3, lequel anticorps est un anticorps monoclonal.

5. Anticorps anti-GM-CSF neutralisant conforme à l'une des revendications 1 à 4 pour l'utilisation de l'une des revendications 1 à 4, lequel anticorps
a) se lie au facteur GM-CSF avec une constante de dissociation K_{D} qui vaut d'environ 5 pM à environ 50 pM ;
b) et/ou est un anticorps recombinant ou chimérique ;
c) et/ou comporte une région variable humaine ou est un anticorps humain.

6. Anticorps anti-GM-CSF neutralisant conforme à l'une des revendications 1 à 5 pour l'utilisation de l'une des revendications 1 à 5, lequel anticorps
a) est un fragment d'anticorps qui est un fragment Fab, Fab', F(ab')₂, scFv ou dAB, lequel fragment d'anticorps est, en option, conjugué à un polyéthylèneglycol ;
b) ou comporte une région constante de chaîne légère humaine et/ou une région constante de chaîne lourde humaine, laquelle région constante de chaîne lourde humaine est, en option, une chaîne gamma.

7. Anticorps anti-GM-CSF neutralisant conforme à l'une des revendications 1 à 6 pour l'utilisation de l'une des revendications 1 à 6, lequel anticorps se lie au même épitope qu'un anticorps chimérique 19/2 comportant une séquence d'acides aminés de région V_{H} présentée en tant que Séquence N° 1 et une séquence d'acides aminés de région V_{L} présentée en tant que Séquence N° 2.

8. Anticorps anti-GM-CSF neutralisant conforme à l'une des revendications 1 à 6 pour l'utilisation de l'une des revendications 1 à 6, lequel anticorps comprend les régions V_{H} et V_{L} d'un anticorps chimérique 19/2 comportant une séquence d'acides aminés de région V_{H} présentée en tant que Séquence N° 1 et une séquence d'acides aminés de région V_{L} présentée en tant que Séquence N° 2.

9. Anticorps anti-GM-CSF neutralisant conforme à la revendication 8 pour l'utilisation de la revendication 8, lequel anticorps comporte une région constante de chaîne lourde humaine.

10. Anticorps anti-GM-CSF neutralisant conforme à la revendication 9 pour l'utilisation de la revendication 9, dans lequel la région constante de chaîne lourde humaine est une région gamma.

11. Anticorps anti-GM-CSF neutralisant conforme à l'une des revendications 1 à 6 pour l'utilisation de l'une des revendications 1 à 6, lequel anticorps
a) comporte les régions CDR1, CDR2 et CDR3 de région V_{H} et de région V_{L} d'un anticorps chimérique 19/2 comportant une séquence d'acides aminés de région V_{H} présentée en tant que Séquence N° 1 et une séquence d'acides aminés de région V_{L} présentée en tant que Séquence N° 2 ;
b) ou comporte la région CDR3 de région V_{H} et la région CDR3 et de région V_{L} d'un anticorps chimérique 19/2 comportant une séquence d'acides aminés de région V_{H} présentée en tant que Séquence N° 1 et une séquence d'acides aminés de région V_{L} présentée en tant que Séquence N° 2.

12. Anticorps anti-GM-CSF neutralisant conforme à l'une des revendications précédentes pour l'utilisation de l'une des revendications précédentes, ledit procédé comportant en outre le fait d'administrer un agent thérapeutique choisi dans l'ensemble formé par un bis-phosphonate, du raloxifène, du tériparatide, du ranélate de strontium, un antagoniste de RANKL, et une protéine de fusion OPG-Fc d'ostéoprotégérine et de fragment Fc.

13. Anticorps anti-GM-CSF neutralisant conforme à la revendication 12 pour l'utilisation de la revendication 12, le bis-phosphonate étant choisi dans l'ensemble constitué par les alendronate, étidronate, risédronate et acide ibandronique.

14. Anticorps anti-GM-CSF neutralisant conforme à la revendication 1 pour l'utilisation de la revendication 1, lequel anticorps
a) comprend un fragment Fab' humain modifié qui présente la spécificité de liaison d'un anticorps chimérique 19/2 comportant une séquence d'acides aminés de région V_{H} présentée en tant que Séquence N° 1 et une séquence d'acides aminés de région V_{L} présentée en tant que Séquence N° 2 ;
b) et se lie au facteur GM-CSF avec une constante de dissociation K_{D} qui vaut d'environ 5 pM à environ 50 pM.

15. Anticorps anti-GM-CSF neutralisant conforme à l'une des revendications précédentes pour l'utilisation de l'une des revendications précédentes, l'ostéopénie résultant d'une carence en oestrogènes.
